# EUROPÄISCHE PATENTSCHRIFT

(11) **EP 3 325 054 B1**
(45) Veröffentlichungstag und Bekanntmachung des Hinweises auf die Patenterteilung: **03.03.2021**
(21) Anmeldenummer: 16732316.1
(22) Anmeldetag: 28.06.2016
(51) Int. Cl.: A61M 5/32, A61M 5/31

(54) **SICHERHEITSVORRICHTUNG FÜR EINE SPRITZE**
SAFETY DEVICE FOR A SYRINGE
DISPOSITIF DE SÉCURITÉ POUR SERINGUE

(30) Priorität: 21.07.2015 DE 102015111840
(43) Veröffentlichungstag der Anmeldung: 30.05.2018
(73) Patentinhaber: Gerresheimer Regensburg GmbH, 93047 Regensburg (DE); Gerresheimer Bünde GmbH, 32257 Bünde (DE)
(72) Erfinder: WITTLAND, Frank, 32130 Enger (DE); VOGL, Maximilian, 92708 Mantel (DE)
(74) Vertreter: Hannke, Christian
(86) Internationale Anmeldenummer: PCT/EP2016/064909
(87) Internationale Veröffentlichungsnummer: WO 2017/012833

(56) Entgegenhaltungen:
- WO-A1-2011/039238
- WO-A1-2014/131987
- US-A1- 2009 198 196
- US-A1- 2015 190 586

## Beschreibung

Die Erfindung betrifft eine Sicherheitsvorrichtung zur Vermeidung von Stichverletzungen für eine Spritze mit einem Spritzenkörper und einem an dem distalen Ende des Spritzenkörpers angeordneten Stechmittel, umfassend ein sich entlang einer axialen Richtung (X) erstreckendes Hülsenelement, welches zumindest teilweise das Stechmittel und den Spritzenkörper umschließt und ein inneres Element, das zumindest abschnittsweise innerhalb des Hülsenelements angeordnet ist und das Stechmittel umschließt.

Gattungsgemäße Sicherheitsvorrichtungen zur Vermeidung von Stichverletzungen sind aus dem Stand der Technik bekannt. Insbesondere bei vorgefüllten Spritzen ist die Verwendung derartiger Sicherheitsvorrichtungen sinnvoll. Die Handhabung derartiger Spritzen ist sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und müssen mit Schutzkappen ausgestattet sein, um Beschädigungen und/oder eine Kontaminierung der Kanüle vor der Anwendung der Spritze zu vermeiden. Darüber hinaus ist es wichtig, nach Gebrauch der Spritze die Kanüle zu sichern, um Stichverletzungen zu vermeiden. Dabei kann ein unvorsichtiges Wiederaufsetzen der Schutzkappe auf die Kanüle Stichverletzungen verursachen. Oft ist die entsprechende Schutzkappe nicht mehr auffindbar oder es wird vergessen, diese wiederaufzusetzen, wodurch ein vermeidbares Verletzungsrisiko gegeben ist.

Demzufolge wurden Nadelschutzeinrichtungen entwickelt, welche fest mit der Spritze verbunden sind und die Nadel nach Verwendung der Spritze automatisch wieder aufnehmen. Eine solche Nadelschutzeinrichtung ist beispielsweise in DE 11 2009 001 083 T5 offenbart. Hierbei wird eine federangetriebene Sicherheitshülse gezeigt, welche in einem ausgefahrenen Zustand die Kanüle umgibt und diese gegen Verletzungen der Anwender sichert. Die Sicherheitshülse weist dabei eine Kurvenbahn auf, in welcher mindestens ein Führungsstift läuft, wodurch verschiedene Positionen der Sicherheitshülse in Abhängigkeit zur Nadelspitze realisiert werden können. Der mindestens eine Führungsstift muss dabei über einen Kragen an der Frontgeometrie der Spritze befestigt werden oder muss auf andere Weise fest mit der Spritze verbunden werden. Der Kragen mit dem Führungsstift darf, um Manipulation oder Fehlbenutzung vorzubeugen, nicht oder nur schwer von der Spritze mit einer Kanüle entfernbar sein. Demnach ist ein entsprechend fester Sitz in axialer Richtung notwendig.

Im Bereich der vorgefüllten Spritzen wird bereits vor dem Abfüllprozess eine Sicherheitsvorrichtung zur Vermeidung von Stichverletzungen beziehungsweise eine Schutzkappe auf dem Spritzenkörper montiert und in einer Standardverpackung, beispielsweise einem Spritzennest, sterilisiert. Man spricht in diesem Zusammenhang auch von "Ready to Use" (RTU)-beziehungsweise "Ready to Sterilize" (RTS)-Spritzen. In der Regel ist neben der Sicherheitsvorrichtung ein weiteres Nadelschutzteil über der Kanüle angeordnet, um die Kanüle vor Beschädigungen und/oder Kontamination zu schützen. Ein solches Nadelschutzteil wird auch Rigid Needle Shield oder Flexible Needle Shield genannt. Die Montage gattungsgemäßer Sicherheitsvorrichtungen mit zusätzlichem Nadelschutzteil gestaltet sich bisher aufwendig. Dies hat hohe Produktionskosten und eine unzureichende Stückzahl hergestellter Sicherheitsvorrichtungen zur Folge.

Aufgabe der vorliegenden Erfindung ist es, eine Sicherheitsvorrichtung zur Vermeidung von Stichverletzungen für eine Spritze zur Verfügung zu stellen, welche eine einfache und kostengünstige Montage ermöglicht.

Diese Aufgabe wird gelöst durch eine Sicherheitsvorrichtung zur Vermeidung von Stichverletzungen, gemäß Anspruch 1. Diese Sicherheitsvorrichtung zur Vermeidung von Stichverletzungen ist für eine Spritze mit einem Spritzenkörper und einem an dem distalen Ende des Spritzenkörpers angeordneten Stechmittel vorgesehen, und umfasst ein sich entlang einer axialen Richtung (X) erstreckendes Hülsenelement, welches zumindest teilweise das Stechmittel und den Spritzenkörper umschließt und ein inneres Element, das zumindest abschnittsweise innerhalb des Hülsenelements angeordnet ist und das Stechmittel umschließt. Die Sicherheitsvorrichtung zeichnet sich weiterhin dadurch aus, dass das innere Element einen Kragenabschnitt und einen das Stechmittel umschließenden Stechmittelschutzabschnitt aufweist, wobei der Kragenabschnitt an einem distalen Endbereich des Spritzenkörpers anordenbar ist und die Sicherheitsvorrichtung an dem Spritzenkörper befestigt und der Stechmittelschutzabschnitt vor der Anwendung der Spritze von dem Kragenabschnitt lösbar ist.

Durch das erfindungsgemäße innere Element wird also sowohl die Sicherheitseinrichtung an dem Spritzenkörper befestigt als auch das Stechmittel von Beschädigungen und/oder Kontamination geschützt. Das Stechmittel kann hier eine Kanüle, eine Nadel oder auch eine Lanzette sein. Im Stand der Technik wird üblicherweise ein Montageelement zur Befestigung der Sicherheitsvorrichtung verwendet und darüber hinaus ein Nadelschutzteil zum Schutz des Stechmittels. Durch das erfindungsgemäße innere Element kann die Endmontage der Sicherheitsvorrichtung sehr einfach vonstatten gehen, da lediglich ein Element montiert wird, welches die Funktion eines Montageelements und eines Nadelschutzteils ausfüllt. Das innere Element ist sozusagen eine einstückige beziehungsweise einteilige Ausführung eines Montagelements und eines Nadelschutzteils. Vorzugsweise kann die Montage des inneren Elements lediglich durch eine Drückbewegung erfolgen. Dies schafft gegenüber dem Stand der Technik einen wesentlichen Vorteil hinsichtlich der Effizienz des Montageprozesses. Vor dem Gebrauch der Spritze kann der Stechmittelschutzabschnitt von dem Kragenabschnitt gelöst werden, wodurch das Stechmittel freigegeben wird.

Gemäß einem besonders bevorzugten Gedanken der Erfindung ist zwischen dem Kragenabschnitt und dem Stechmittelschutzabschnitt eine Sollbruchstelle angeordnet. Vor Anwendung der Spritze kann, beispielsweise durch eine Dreh- oder eine Ziehbewegung, die Sollbruchstelle aufgebrochen werden und der Stechmittelschutzabschnitt von dem Kragenabschnitt gelöst werden.

Vorzugsweise ist die Sollbruchstelle zwischen einer distalen Stirnfläche des Kragenabschnitts und einem proximalen Ende des Stechmittelschutzabschnitts angeordnet. Bevorzugt ist die Sollbruchstelle eine Materialverjüngung und/oder eine Perforation und/oder eine Einkerbung und/oder ein Ritz in dem Material. Vorstellbar sind aber auch anderweitige bekannte Ausführungsformen von Sollbruchstellen.

Nach einem weiteren besonders bevorzugten Gedanken der Erfindung weist der Kragenabschnitt zumindest einen Führungsvorsprung auf, welcher in zumindest einer Führungskulisse des Hülsenelements eingreift. Vorzugsweise ist der Führungsvorsprung in der zumindest einen Führungskulisse es Hülsenelements bei einer Relativbewegung des Spritzenkörpers zu dem Hülsenelement im Wesentlichen entlang der axialen Richtung (X) geführt. Die Sicherheitsvorrichtung ist dabei im Wesentlichen in Form eines hohlen Kreiszylinders ausgestaltet. Bei einer Anwendung der Spritze wird die Spritze mit der Sicherheitseinrichtung gegen die Haut des Patienten gedrückt. Durch die Relativbewegung des Spritzenkörpers zu dem Hülsenelement und die Führung des Führungsvorsprungs in der Führungskulisse, welche eine Kurvenbahn aufweist, wird eine Rotation des Hülsenelements entlang einer Umfangsrichtung (U) verursacht. Das Hülsenelement schiebt sich dadurch vorzugsweise über den Spritzenkörper, wodurch das Stechmittel, welches eine Kanüle, eine Nadel oder auch eine Lanzette sein kann, durch eine entsprechende Öffnung in dem Hülsenelement hindurchtritt.

Gemäß einer bevorzugten Ausführungsform ist der Kragenabschnitt in einer Umfangsrichtung (U) rotierbar an dem distalen Endbereich des Spritzenkörpers angeordnet. Demnach wird bei einer Anwendung der Spritze durch die Relativbewegung des Spritzenkörpers zu dem Hülsenelement und die Führung des Führungsvorsprungs in der Führungskulisse eine Rotationsbewegung des Kragenabschnitts anstatt des Hülsenelements entlang einer Umfangsrichtung (U) verursacht. Das Hülsenelement schiebt sich dadurch vorzugsweise über den Spritzenkörper, wodurch das Stechmittel durch eine entsprechende Öffnung in dem Hülsenelement hindurchtritt. Somit wird eine bei dem Patienten als unangenehm empfunden Rotation des Hülsenelements auf dessen Haut um die Einstichstelle herum vermieden.

Vorzugsweise weist der Stechmittelschutzabschnitt in einem proximalen Endbereich einen Hohlraum auf. Bevorzugt sind in dem Hohlraum das Stechmittel und abschnittsweise der distale Endbereich des Spritzenkörpers anordenbar.

Bevorzugt ist in dem Hohlraum des Stechmittelschutzabschnitts ein Innenteil aus einem elastischen Material angeordnet. Darüber hinaus ist das elastische Material bevorzugt Gummi oder ein synthetischer Elastomer. Vorteilhafterweise umschließt das Innenteil dabei das Stechmittel. In der Regel weisen Stechmittel, beispielsweise Kanülen, einen sehr feinen Schliff auf, um eine möglichst schmerzfreie Injektion zu ermöglichen. Solche Schliffe können leicht durch mechanische Einflüsse beschädigt werden, wodurch dem Patienten bei einer Injektion unnötige Schmerzen bereitet werden können. Durch das elastische Innenteil wird ein weiterer Schutz gegenüber mechanischen Einflüssen bereitgestellt. Das Stechmittel liegt somit vorteilhaft an den Innenwänden des elastischen Materials an, wodurch bei mechanischen Einflüssen der Schliff geschützt wird. Ferner wäre es denkbar, dass das Innenteil mit einem proximalen Ende dichtend an dem distalen Endbereich des Spritzenkörpers anliegt. Der das Stechmittel einschließende Bereich wäre somit dichtend abgeschlossen. Dies bewirkt einen wirksamen Schutz des Stechmittels vor Kontamination.

Nach einem weiteren vorteilhaften Gedanken der Erfindung ist der Kragenabschnitt im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest eine Führungsvorsprung radial von der Mantelfläche weg. Weiterhin bevorzugt ist der Führungsvorsprung als Kreiszylinder beziehungsweise als Stift ausgebildet. Vorteilhafterweise sind an der Mantelfläche zwei sich diametral gegenüberliegende Führungsvorsprünge angeordnet. Demzufolge würde auch das Hülsenelement zwei sich diametral gegenüberliegende Führungskulissen aufweisen, in denen jeweils ein Führungsvorsprung geführt wird. Weiter bevorzugt ist ein Außendurchmesser des Kragenabschnitts größer als ein Außendurchmesser des Stechmittelschutzabschnitts.

Vorzugsweise ist der Spritzenkörper als hohler Kreiszylinder ausgestaltet und weist in seinem distalen Endbereich ein konisches Endstück auf, an welchem das Stechmittel angeordnet ist. Vorzugsweise besteht der Spritzenkörper aus Glas oder aus einem Polymer-Kunststoff, bevorzugt einem Polyolefin, beispielsweise Polypropylen oder Polyethylen, besonders bevorzugt aus einem Cyclo-Olefinen Polymer (COP) beziehungsweise aus einem Cyclo-Olefinen Co-Polymer (COC).

Weiter bevorzugt ist an dem konischen Endstück ein Vorsprung ausgebildet, an welchem eine Stirnfläche des distalen Endes des Kragenelements eingreifbar ist, wodurch das Kragenelement und somit die Sicherheitsvorrichtung in axialer Richtung arretierbar ist. Um Manipulation oder Fehlbenutzung der Spritze vorzubeugen, muss die Sicherheitsvorrichtung nicht oder nur schwer von der Spritze mit einer Kanüle entfernbar sein. Demnach ist ein entsprechend fester Sitz in axialer Richtung notwendig.

Vorzugsweise umfasst der Kragenabschnitt einen distalen Bereich und einen proximalen Bereich. Bevorzugt weist in dem distalen Bereich die Wandung des Kragenabschnitts zumindest zwei Schlitze auf, welche sich in axialer Richtung (X) erstrecken. Durch derartige Schlitze ist eine Anpassung des Kragenabschnitts an unterschiedliche Spritzenkörperformen beziehungsweise Spritzenkörperdurchmesser gewährleistet. Ferner ist durch die Schlitze das Aufbringen des inneren Elements auf den Spritzenkörper erleichtert. Weiter bevorzugt weist der Kragenabschnitt in seinem proximalen Bereich einen Innenkonus auf. Durch diesen proximalen Bereich wird ein zweiter Lagerpunkt für den Kragenabschnitt an dem Spritzenkörper im Bereich der Spritzenschultern realisiert.

Vorzugsweise weist die Sicherheitsvorrichtung mindestens ein Federelement auf, das mit dem Spritzenkörper wirkverbunden ist und der Relativbewegung des Spritzenkörpers zu der Sicherheitsvorrichtung entgegenwirkt. Demnach bleibt die Kanüle bis zur vorgesehenen Anwendung innerhalb des Hülsenelements. Bei der Anwendung muss das Hülsenelement gegen die Federkraft verschoben werden, damit das Stechmittel durch die Öffnung des Hülsenelements hindurchtreten kann. Nach Gebrauch der Spritze schiebt sich automatisch, angetrieben durch die Federkraft des Federelements, das Hülsenelement wieder über das Stechmittel. Der Anwender ist somit vor Stichverletzungen mit dem gebrauchten kontaminierten Stechmittel geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Gemäß einer bevorzugten Ausführungsform sind der Kragenabschnitt und der Stechmittelschutzabschnitt aus unterschiedlichen Materialien hergestellt. Vorzugsweise wird das innere Element durch ein Mehrkomponenten- Spritzgussverfahren hergestellt. Bei einem solchen Verfahren weist die verwendete Spritzgießmaschine lediglich ein Spritzgießwerkzeug mit mehreren Spritzeinheiten auf. Die Spritzgussteile können damit kostengünstig mit nur einem Werkzeug in einem Arbeitsgang hergestellt werden. Es wäre auch denkbar, dass die beiden Abschnitte des inneren Elements einzeln hergestellt werden und im Nachhinein verschweißt werden.

Bevorzugt ist der Stechmittelschutzabschnitt aus einem thermoplastischen Elastomer (TPE) und der Kragenabschnitt aus Polyoxymethylen (POM) hergestellt. POM wird wegen seiner hohen Steifigkeit, niedrigen Reibwerte, ausgezeichneten Dimensionsstabilität und thermischen Stabilität als technischer Kunststoff, besonders für Präzisionsteile, eingesetzt. Thermoplastische Elastomere sind Kunststoffe, die sich bei Raumtemperatur vergleichbar den klassischen Elastomeren verhalten, sich jedoch unter Wärmezufuhr plastisch verformen lassen und somit ein thermoplastisches Verhalten zeigen.

Die Aufgabe der Erfindung wird in einem weiteren Aspekt auch gelöst durch ein Verfahren zur Montage der Sicherheitsvorrichtung nach einem der vorhergehenden Ansprüche und Anordnen der Sicherheitsvorrichtung auf einem Spritzenkörper durch folgende Verfahrensschritte:
a) Einbringen des Federelements entlang der axialen Richtung (X) in das Innere des Hülsenelements;
b) Einbringen des inneren Elements entlang der axialen Richtung (X) in das Innere des Hülsenelements, so dass das Federelement zwischen dem Kragenabschnitt des inneren Elements und dem Hülsenelement angeordnet ist;
c) Aufbringen des Kragenabschnitts des inneren Elements auf den distalen Endbereich des Spritzenkörpers;
wobei das innere Element bei der Montage zumindest teilweise verformbar ist.

Beim Aufbringen des inneren Elements, welches einen Kragenabschnitt und einen Stechmittelschutzabschnitt umfasst, auf den Spritzenkörper wird der Kragenabschnitt auf den Spritzenkörper geschoben. Weist nun der Spritzenkörper einen Vorsprung oder eine Verdickung auf, welche beispielsweise zur Arretierung des Kragenabschnitts in axialer Richtung dienen kann, kann ein Aufbringen erschwert werden. Durch die vorteilhaften Schlitze in dem Kragenabschnitt ist jedoch eine geringe Aufweitung des Kragenabschnitts möglich, wodurch dieses leichter auf den Spritzenkörper aufgeschoben werden kann. Eine solche Aufweitung hat eine mechanische Belastung der Sollbruchstelle zwischen dem Kragenabschnitt und dem Stechmittelschutzabschnitt zur Folge. Um eine solche mechanische Belastung zu reduzieren, ist es vorteilhaft, wenn der Stechmittelschutzabschnitt geringfügig verformbar ist. Bevorzugt ist dabei der Stechmittelschutzabschnitt aus einem Kunststoff mit einem thermoplastischen Verhalten hergestellt. Der Kragenabschnitt besteht bevorzugt aus einem Kunststoff, welcher kein solches thermoplastisches Verhalten aufweist. Demnach kann das innere Element vor oder bei Aufschieben auf den Spritzenkörper erwärmt werden, so dass lediglich der Stechmittelschutzabschnitt geringfügig verformbar ist. Somit kann das innere Element auf den Spritzenkörper aufgebracht werden, ohne dass die Sollbruchstelle eine mechanische Belastung erfährt.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: eine isometrische Ansicht einer Spritze mit Sicherheitsvorrichtung;
- Fig.2: eine Schnittdarstellung einer Spritze mit Sicherheitsvorrichtung gemäß einer Ausführungsform;
- Fig.3: eine Schnittdarstellung des inneren Elements gemäß einer Ausführungsform;
- Fig.4: eine Schnittdarstellung des inneren Elements gemäß einer weiteren Ausführungsform.

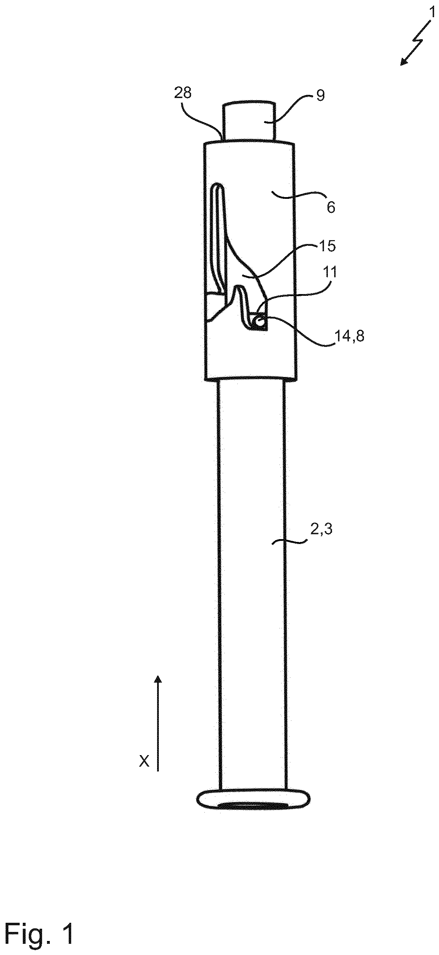

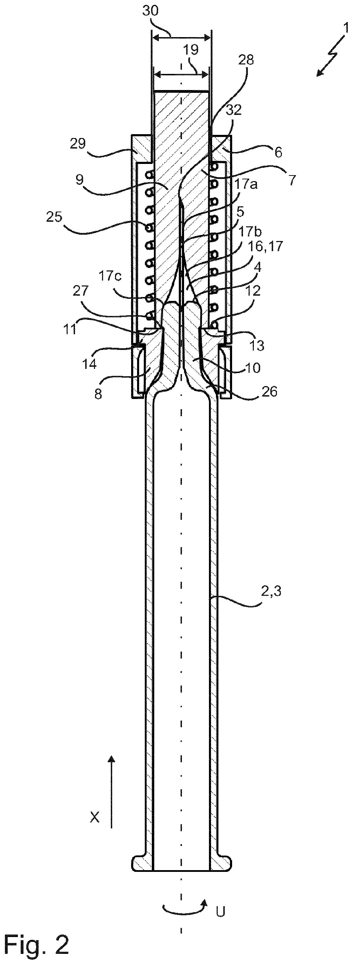

In den Figuren 1 und 2 ist eine Spritze (2) mit einer Sicherheitsvorrichtung (1) zur Vermeidung von Stichverletzungen gezeigt, wobei Fig. 1 eine isometrische Ansicht und Fig. 2 eine Schnittdarstellung zeigen. Die Spritze (2) umfasst einen Spritzenkörper (3), welcher als hohler Kreiszylinder ausgestaltet ist. Der Spritzenkörper weist einen distalen Endbereich (10) mit einem distalen Ende (4) auf. An dem distalen Ende (4) ist ein Stechmittel (5) angeordnet. Dieses Stechmittel (5) ist über eine Bohrung in dem distalen Endbereich (10) mit dem Hohlraum des Spritzenkörpers (3) verbunden, so dass das zu injizierende Medium bei einer Anwendung der Spritze (2) aus dem Hohlraum durch das Stechmittel (5) treten kann. Der distale Endbereich (10) ist als konisches Endstück ausgestaltet, welches einen kleineren Außendurchmesser als der Spritzenkörper (3) aufweist. Weiterhin weist die Spritze einen Übergangsbereich (26) auf, in dem der Außendurchmesser des Spritzenkörpers (3) in den Außendurchmesser des Endstücks übergeht. Darüber hinaus ist an dem distalen Endbereich ein Vorsprung (27) angeordnet.

Weiterhin ist eine Sicherheitsvorrichtung (1) zur Vermeidung von Stichverletzungen für eine Spritze (2) gezeigt, welche einen Spritzenkörper (3) und ein an dem distalen Ende (4) des Spritzenkörpers (3) angeordnetes Stechmittel (5) umfasst. Die Sicherheitsvorrichtung (1) umfasst ein sich entlang einer axialen Richtung (X) erstreckendes Hülsenelement (6), welches zumindest teilweise das Stechmittel (5) und den Spritzenkörper (3) umschließt und ein inneres Element (7), das zumindest abschnittsweise innerhalb des Hülsenelements (6) angeordnet ist und das Stechmittel (5) umschließt. Das innere Element (7) weist einen Kragenabschnitt (8) und einen das Stechmittel (5) umschließenden Stechmittelschutzabschnitt (9) auf, wobei der Kragenabschnitt (8) an einem distalen Endbereich (10) des Spritzenkörpers (3) anordenbar ist und die Sicherheitsvorrichtung (1) an dem Spritzenkörper (3) befestigt und der Stechmittelschutzabschnitt (9) vor der Anwendung der Spritze (2) von dem Kragenabschnitt (8) lösbar ist.

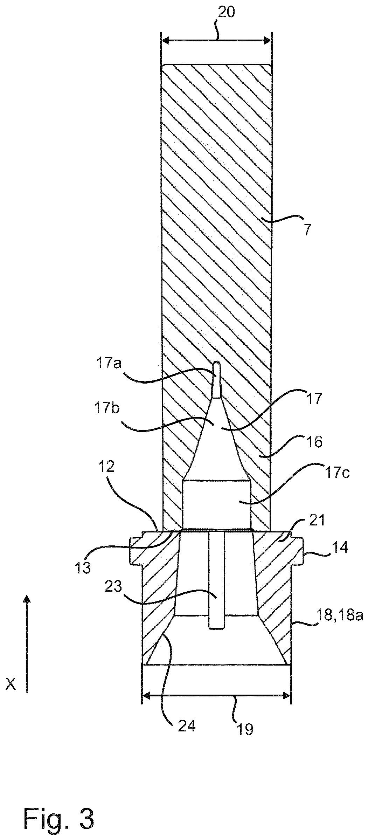

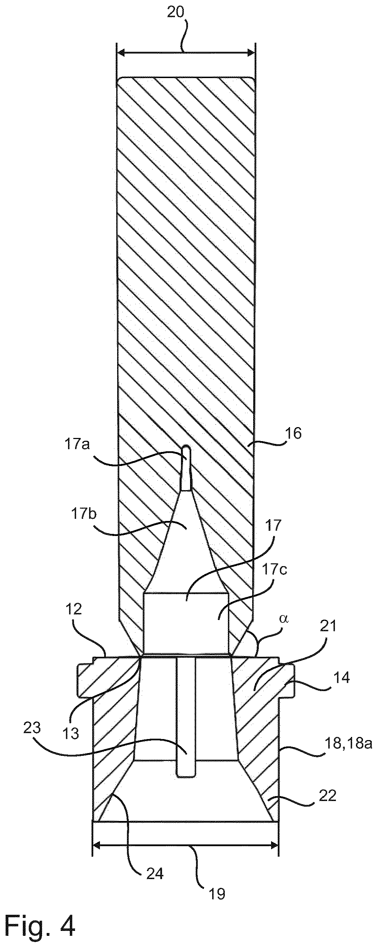

Zwischen dem Kragenabschnitt (8) und dem Stechmittelschutzabschnitt (9) ist eine Sollbruchstelle (11) angeordnet. Die Sollbruchstelle (11) ist zwischen einer distalen Stirnfläche (12) des Kragenabschnitts (8) und einem proximalen Ende (13) des Stechmittelschutzabschnitts (9) angeordnet. Eine detaillierte Ansicht des inneren Elements gemäß zwei verschiedenen Ausführungsformen ist in den Figuren 3 und 4 gezeigt. Die Sollbruchstelle (11) kann eine Materialverjüngung und/oder eine Perforation und/oder eine Einkerbung und/oder ein Ritz in dem Material sein. In Fig. 4 ist beispielsweise eine solche Materialverjüngung gezeigt. In einem Abschnitt des proximalen Endbereichs (16) des Stechmittelschutzabschnitts (9) verjüngt sich dabei der Außendurchmesser (19) des Stechmittelschutzabschnitts (9) in axialer Richtung (X) zu dem Kragenabschnitt (8) hin. Dadurch, dass der Innendurchmesser des Hohlraums (17) in diesem Bereich konstant bleibt, verringert sich die Wandstärke in diesem Bereich, wodurch eine Sollbruchstelle (11) geschaffen wird. Durch die lineare Abnahme des Außendurchmessers (19) schließen die distale Stirnfläche (12) des Kragenabschnitts (8) und eine Außenfläche des Stechmittelschutzabschnitts (9) einen Winkel α ein. Der Winkel α liegt bevorzugt in einem Bereich zwischen 30° und 60°, besonders bevorzugt bei 45°. In Fig. 3 wird eine weitere Ausführungsform des inneren Elements (7) gezeigt. Die Sollbruchstelle (11) ist hierbei eine Perforation und/oder ein Ritz in dem Material.

Der Kragenabschnitt (8) ist im Wesentlichen als hohler Kreiszylinder (18) ausgebildet. Der Kreiszylinder (18) weist eine Mantelfläche (18a) auf, an der zwei Führungsvorsprünge (14) angeordnet sind. Die Führungsvorsprünge (14) erstrecken sich radial von der Mantelfläche (18a) nach außen weg und sind diametral gegenüberliegend angeordnet. Weiterhin sind diese als Kreiszylinder beziehungsweise als Stift ausgebildet. Diese beiden Führungsvorsprünge (14) sind jeweils in einer Führungskulisse (15) des Hülsenelements (6) bei einer Relativbewegung des Spritzenkörpers (3) zu dem Hülsenelement (6) im Wesentlichen entlang der axialen Richtung (X) geführt. Ferner ist der Kragenabschnitt (8) in einer Umfangsrichtung (U) rotierbar an dem distalen Endbereich (10) des Spritzenkörpers (3) angeordnet.

Der Kragenabschnitt (8) umfasst weiterhin einen distalen Bereich (21) und einen proximalen Bereich (22). In dem distalen Bereich weist der Kragenabschnitt (8) eine distale Stirnfläche (12) auf. Ein Abschnitt dieser distalen Stirnfläche liegt an einem Vorsprung (27) bzw. einer Verdickung an dem distalen Ende (4) des Spritzenkörpers (3) an, wodurch die Arretierung in axialer Richtung (X) ermöglicht ist. Ein weiterer Abschnitt dieser distalen Stirnfläche (12) bildet dem proximalen Ende (13) des Stechmittelschutzabschnitts (9) die Sollbruchstelle (11). Schließlich liegt gemäß einer Ausführungsform an einem noch weiteren Abschnitt ein Federelement (25) an. Die Wandung des Kragenabschnitts (8) weist in dem distalen Bereich (21) weiterhin zwei Schlitze (23) auf. Die Schlitze (23) erstrecken sich ausgehend von der distalen Stirnfläche (12) in axialer Richtung (X). Durch die Schlitze (23) wird eine bessere Anpassung des Kragenabschnitts (8) beziehungsweise des inneren Elements (7) an unterschiedliche Spritzenkörper (3) beziehungsweise ein leichteres Aufbringen des inneren Elements (7) auf den Spritzenkörper (3) ermöglicht. Üblicherweise wird das innere Element (7) über das distale Ende (4) des Spritzenkörpers geschoben. Dieses distale Ende (4) weist einen Vorsprung (27) beziehungsweise eine Verdickung auf. Durch die Schlitze (23) kann der Kragenabschnitt (8) während des Aufbringens aufgeweitet werden, wodurch dieser leichter über den Vorsprung (27) geschoben werden kann. Um mechanische Belastungen auf die Sollbruchstelle (11) zu vermeiden, ist es vorteilhaft, wenn der Stechmittelschutzabschnitt (9) während des Aufbringens des inneren Elements (7) auf den Spritzenkörper (3) zumindest geringfügig verformbar ist. Der Kragenabschnitt (8) weist weiterhin in seinem proximalen Bereich (22) einen Innenkonus (24) auf. Dieser Innenkonus (24) liegt an dem Übergangsbereich (26) des Spritzenkörpers (3) an und dient somit als zweiter Lagerpunkt.

Das distale Ende (32) und ein anschließender distaler Bereich des Stechmittels (5) sind in einem Hohlraum (17) in einem proximalen Endbereich (16) des Stechmittelschutzabschnitts (9) angeordnet. Der Hohlraum umfasst einen ersten Bereich (17a), wobei das Stechmittel (5) in diesem ersten Bereich an dessen Innenwänden anliegend ist. Vorteilhafterweise kann in dem Hohlraum (17) des Stechmittelschutzabschnitts (9) ein Innenteil aus einem elastischen Material, beispielsweise Gummi oder einem synthetischen Elastomer angeordnet sein. Das Stechmittel (5) beziehungsweise der Schliff des Stechmittels wäre somit gegen Beschädigungen durch mechanische Einflüsse geschützt. In einem dritten Bereich (17c) erstreckt sich der Hohlraum (17) weiterhin über das distale Ende (4) bzw. einen Teil des distalen Endbereichs (10) des Spritzenkörpers (3). Zwischen dem ersten (17a) und dem dritten Bereich (17c) befindet sich ein zweiter Bereich (17b), in dem sich der Innendurchmesser des Hohlraums (17) ausgehend von dem ersten Bereich (17a) bis zu dem dritten Bereich (17c) vergrößert. Vorteilhafterweise liegt die Innenwand des Stechmittelschutzabschnitts beziehungsweise das Innenteil in dem dritten Bereich (17c) dichtend an dem distalen Ende (4) des Spritzenkörpers (3) an. Somit ist das Stechmittel (5) vor Kontamination geschützt.

In Fig. 2 ist weiterhin eine Sicherheitsvorrichtung (1) dargestellt, welche ein Federelement (25) in Form einer Spiralfeder aufweist, das mit dem Spritzenkörper (3) wirkverbunden ist und der Relativbewegung des Hülsenelements (6) zu der Sicherheitsvorrichtung (1) entgegenwirkt. Das innere Element (7) ist innerhalb des Hülsenelements (6) angeordnet. Das Federelement umschließt dabei den Stechmittelschutzabschnitt (9). Ein Außendurchmesser (19) des Kragenabschnitts (8) ist größer als ein Außendurchmesser (20) des Stechmittelschutzabschnitts (9). Somit liegt das Federelement (25) einerseits an einer distalen Stirnfläche (12) des Kragenabschnitts (8) und anderseits an einem die distale Öffnung (28) des Hülsenelements (6) begrenzenden Wandungsbereich (29) an. Der Außendurchmesser (19) des Stechmittelschutzabschnitts (9) ist weiterhin kleiner als ein Innendurchmesser (30) der distalen Öffnung (28) des Hülsenelements (6). Der Stechmittelschutzabschnitt (9) erstreckt sich durch die distale Öffnung (28) und ragt somit über das Hülsenelement hinaus. Der über das Hülsenelement hinausragende Bereich dient als Griffbereich (31), durch welchen eine Dreh- oder Zugkraft auf das innere Element (7), beziehungsweise die Sollbruchstelle (11) ausgeübt werden kann. Denkbar wäre es, die Oberfläche des inneren Elements (7) in diesem Griffbereich (31) so auszugestalten, dass diese rutschhemmend wirkt. Dies könnte beispielsweise durch eine entsprechende Beschichtung oder eine aufgeraute Oberfläche erfolgen.

Nach dem Aufbrechen der Sollbruchstelle, beispielsweise durch eine Dreh- oder Ziehbewegung und Entfernen des Stechmittelschutzabschnitts (9) bleibt das Stechmittel (5) bis zur vorgesehenen Anwendung innerhalb des Hülsenelements (6). Bei einer Anwendung wird die Spritze (2) beziehungsweise die Sicherheitsvorrichtung (1) mit ihrem distalen Ende an die Haut des Patienten gepresst. Bei der Anwendung wird dabei das Hülsenelement (6) gegen die Federkraft des Federelements (25) verschoben, damit das Stechmittel (5) durch die distale Öffnung (28) des Hülsenelements (6) hindurchtreten kann. Das Hülsenelement (6) wird dabei über den distalen Endbereich (10) des Spritzenkörpers (3) geschoben. Durch die Führung des Führungsvorsprungs (14) in der Führungskulisse (15) rotiert der Kragenabschnitt (8) entlang der Umfangsrichtung (U). Nach Gebrauch der Spritze (2) schiebt sich automatisch, angetrieben durch die Federkraft des Federelements (25), das Hülsenelement (6) wieder über das Stechmittel (5). Durch die Führung der Führungsvorsprünge (14) in den Führungskulissen (15) rotiert der Kragenabschnitt (8) entgegen der Umfangsrichtung (U). Der Anwender ist somit vor Stichverletzungen mit dem gebrauchten und kontaminierten Stechmittel geschützt.

### Bezugszeichenliste

- 1: Sicherheitsvorrichtung
- 2: Spritze
- 3: Spritzenkörper
- 4: distales Ende des Spritzenkörpers
- 5: Stechmittel
- 6: Hülsenelement
- 7: inneres Element
- 8: Kragenabschnitt
- 9: Stechmittelschutzabschnitt
- 10: distaler Endbereich
- 11: Sollbruchstelle
- 12: distale Stirnfläche des Kragenabschnitts
- 13: proximales Ende des Stechmittelschutzabschnitts
- 14: Führungsvorsprung
- 15: Führungskulisse
- 16: proximaler Endbereich des Stechmittelschutzabschnitts
- 17: Hohlraum
- 17a: erster Bereich des Hohlraums
- 17b: zweiter Bereich des Hohlraums
- 17c: dritter Bereich des Hohlraums
- 18: Kreiszylinder
- 18a: Mantelfläche es Kreiszylinders
- 19: Außendurchmesser des Kragenabschnitts
- 20: Außendurchmesser des Stechmittelschutzabschnitts
- 21: distaler Bereich des Kragenabschnitts
- 22: proximaler Bereich des Kragenabschnitts
- 23: Schlitze
- 24: Innenkonus
- 25: Federelement
- 26: Übergangsbereich
- 27: Vorsprung
- 28: distale Öffnung des Hülsenelements
- 29: Wandungsbereich
- 30: Innendurchmesser
- 31: Griffbereich
- 32: distales Ende des Stechmittels
- X: axiale Richtung
- U: Umfangsrichtung

## Patentansprüche

1. Sicherheitsvorrichtung (1) zur Vermeidung von Stichverletzungen für eine Spritze (2) mit einem Spritzenkörper (3) und einem an dem distalen Ende (4) des Spritzenkörpers (3) angeordneten Stechmittel (5), umfassend ein sich entlang einer axialen Richtung (X) erstreckendes Hülsenelement (6), welches zumindest teilweise das Stechmittel (5) und den Spritzenkörper (3) umschließt und ein inneres Element (7), das zumindest abschnittsweise innerhalb des Hülsenelements (6) angeordnet ist und das Stechmittel (5) umschließt, wobei das innere Element (7) einen Kragenabschnitt (8) und einen das Stechmittel (5) umschließenden Stechmittelschutzabschnitt (9) aufweist, wobei der Kragenabschnitt (8) in einer Umfangsrichtung (U) rotierbar an einem distalen Endbereich (10) des Spritzenkörpers (3) angeordnet ist und die Sicherheitsvorrichtung (1) an dem Spritzenkörper (3) befestigt,
**dadurch gekennzeichnet, dass**
zwischen dem Kragenabschnitt (8) und dem Stechmittelschutzabschnitt (9) eine Sollbruchstelle (11) angeordnet ist, wodurch der Stechmittelschutzabschnitt (9) vor Anwendung der Spritze (2) von dem Kragenabschnitt (8) lösbar ist, wobei der Kragenabschnitt (8) zumindest einen Führungsvorsprung (14) aufweist, welcher in zumindest einer eine Kurvenbahn aufweisende Führungskulisse (15) des Hülsenelements (6) eingreift, wobei die Sollbruchstelle (11) zwischen einer distalen Stirnfläche (12) des Kragenabschnitts (8) und einem proximalen Ende (13) des Stechmittelschutzabschnitts (9) angeordnet ist, wobei der Kragenabschnitt (8) und der Stechmittelschutzabschnitt (9) das innere Element (7) einstückig ausbilden.

2. Sicherheitsvorrichtung (1) nach Anspruch 1,
**dadurch gekennzeichnet, dass**
die Sollbruchstelle (11) eine Materialverjüngung und/oder eine Perforation und/oder eine Einkerbung und/oder ein Ritz in dem Material ist.

3. Sicherheitsvorrichtung (1) nach einem der vorhergehenden Ansprüche,
**dadurch gekennzeichnet, dass**
der Führungsvorsprung (14) in der zumindest einen Führungskulisse (15) des Hülsenelements (6) bei einer Relativbewegung des Spritzenkörpers (3) zu dem Hülsenelement (6) im Wesentlichen entlang der axialen Richtung (X) geführt ist.

4. Sicherheitsvorrichtung (1) nach einem der vorhergehenden Ansprüche,
**dadurch gekennzeichnet, dass**
der Stechmittelschutzabschnitt (9) in einem proximalen Endbereich (16) einen Hohlraum (17) aufweist, wobei in dem Hohlraum (17) das Stechmittel (5) und abschnittsweise der distale Endbereich (10) des Spritzenkörpers (3) anordenbar sind.

5. Sicherheitsvorrichtung (1) nach Anspruch 4,
**dadurch gekennzeichnet, dass**
in dem Hohlraum (17) des Stechmittelschutzabschnitts (9) ein Innenteil aus einem elastischen Material angeordnet ist, wobei das elastische Material Gummi oder ein synthetischer Elastomer ist.

6. Sicherheitsvorrichtung (1) nach einem der vorhergehenden Ansprüche,
**dadurch gekennzeichnet, dass**
der Kragenabschnitt (8) im Wesentlichen als hohler Kreiszylinder (18) ausgebildet ist, wobei der Kreiszylinder (18) eine Mantelfläche (18a) aufweist, an der der zumindest eine Führungsvorsprung (14) angeordnet ist, wobei ein Außendurchmesser (19) des Kragenabschnitts (8) größer ist als ein Außendurchmesser (20) des Stechmittelschutzabschnitts (9).

7. Sicherheitsvorrichtung (1) nach einem der vorhergehenden Ansprüche,
**dadurch gekennzeichnet, dass**
der Kragenabschnitt (8) einen distalen Bereich (21) und einen proximalen Bereich (22) umfasst, wobei in dem distalen Bereich (21) die Wandung des Kragenabschnitts (8) zumindest zwei Schlitze (23) aufweist, welche sich in axialer Richtung (X) erstrecken, und wobei der proximale Bereich (22) einen Innenkonus (24) aufweist.

8. Sicherheitsvorrichtung (1) nach einem der vorhergehenden Ansprüche,
**dadurch gekennzeichnet, dass**
die Sicherheitsvorrichtung (1) mindestens ein Federelement (25) aufweist, das mit dem Spritzenkörper (3) wirkverbunden ist und der Relativbewegung des Spritzenkörpers (3) zu dem Hülsenelement (6) entgegenwirkt.

9. Sicherheitsvorrichtung (1) nach einem der vorhergehenden Ansprüche,
**dadurch gekennzeichnet, dass**
der Kragenabschnitt (8) und der Stechmittelschutzabschnitt (9) aus unterschiedlichen Materialien hergestellt sind.

10. Sicherheitsvorrichtung (1) nach einem der vorhergehenden Ansprüche,
**dadurch gekennzeichnet, dass**
der Stechmittelschutzabschnitt (9) aus einem thermoplastischen Elastomer (TPE) und der Kragenabschnitt aus Polyoxymethylen (POM) hergestellt ist.

11. Verfahren zur Montage der Sicherheitsvorrichtung (1) nach Anspruch 8 und Anordnen der Sicherheitsvorrichtung (1) auf einem Spritzenkörper (3) durch folgende Verfahrensschritte:
a) Einbringen des Federelements (25) entlang der axialen Richtung (X) in das Innere des Hülsenelements (6);
b) Einbringen des inneren Elements (7) entlang der axialen Richtung (X) in das Innere des Hülsenelements (6), so dass das Federelement (25) zwischen dem Kragenabschnitt (8) des inneren Elements (7) und dem Hülsenelement (6) angeordnet ist;
c) Aufbringen des Kragenabschnitts (8) des inneren Elements (7) auf den distalen Endbereich (10) des Spritzenkörpers (3);
wobei das innere Element (7) bei der Montage erwärmt wird, so dass dieses zumindest teilweise verformbar ist.

## Claims

1. Safety device (1) for a syringe (2) for avoiding stab wounds, said syringe having a syringe body (3) and a piercing means (5) arranged at the distal end (4) of the syringe body (3), said safety device comprising a sleeve element (6) which extends in an axial direction (X) and encloses the piercing means (5) and the syringe body (3) at least in part, and an inner element (7), at least portions of which are arranged inside the sleeve element (6) and enclose the piercing means (5),
wherein the inner element (7) comprises a collar portion (8) and a piercing means protection portion (9) that encloses the piercing means (5), the collar portion (8) being arranged on a distal end region (10) of the syringe body (3) such that it can rotate in a circumferential direction (U) and fastening the safety device (1) to the syringe body (3),
**characterised in that**
a predetermined breaking point (11) being arranged between the collar portion (8) and the piercing means protection portion (9), as a result of which the piercing means protection portion (9) can be detached from the collar portion (8) before the syringe (2) is used, wherein the collar portion (8) has at least one guide projection (14) which engages in at least one guide slot (15) of the sleeve element (6), the at least one guide slot having a curved path, wherein the predetermined breaking point (11) is arranged between a distal end face (12) of the collar portion (8) and a proximal end (13) of the piercing means protection portion (9), wherein the collar portion (8) and the piercing means protection portion (9) form the inner element (7) in one piece.

2. Safety device (1) according to claim 1,
**characterised in that**
the predetermined breaking point (11) being a material taper and/or a perforation and/or a notch and/or a score in the material.

3. Safety device (1) according to any of the preceding claims,
**characterised in that**
the guide projection (14) being substantially guided in the axial direction (X) in the at least one guide slot (15) of the sleeve element (6) when the syringe body (3) moves relative to the sleeve element (6).

4. Safety device (1) according to any of the preceding claims,
**characterised in that**
the piercing means protection portion (9) has a cavity (17) in a proximal end region (16), the piercing means (5) and portions of the distal end region (10) of the syringe body (3) being arrangeable in the cavity (17).

5. Safety device (1) according to claim 4,
**characterised in that**
an inner part made of a resilient material is arranged in the cavity (17) in the piercing means protection portion (9), the resilient material being rubber or a synthetic elastomer.

6. Safety device (1) according to any of the preceding claims,
**characterised in that**
the collar portion (8) is substantially formed as a hollow circular cylinder (18), the circular cylinder (18) having a lateral surface (18a) on which the at least one guide projection (14) is arranged, an external diameter (19) of the collar portion (8) being greater than an external diameter (20) of the piercing means protection portion (9).

7. Safety device (1) according to any of the preceding claims,
**characterised in that**
the collar portion (8) comprises a distal region (21) and a proximal region (22), in the distal region (21) the wall of the collar portion (8) having at least two slits (23) which extend in the axial direction (X), and the proximal region (22) having an inside taper (24).

8. Safety device (1) according to any of the preceding claims,
**characterised in that**
the safety device (1) has at least one spring element (25), which is operatively connected to the syringe body (3) and counteracts the movement of the syringe body (3) relative to the sleeve element (6).

9. Safety device (1) according to any of the preceding claims,
**characterised in that**
the collar portion (8) and the piercing means protection portion (9) are made of different materials.

10. Safety device (1) according to any of the preceding claims,
**characterised in that**
the piercing means protection portion (9) is made of a thermoplastic elastomer (TPE) and the collar portion is made of polyoxymethylene (POM).

11. Method for mounting the safety device (1) according to claim 8 and for arranging the safety device (1) on a syringe body (3) using the following method steps:
a) inserting the spring element (25) into the interior of the sleeve element (6) in the axial direction (X);
b) inserting the inner element (7) into the interior of the sleeve element (6) in the axial direction (X) such that the spring element (25) is arranged between the collar portion (8) of the inner element (7) and the sleeve element (6); and
c) attaching the collar portion (8) of the inner element (7) to the distal end region (10) of the syringe body (3);
wherein the inner element (7) is heated during the mounting process so as to be at least partially deformable.

## Revendications

1. Dispositif de sécurité (1), destiné à éviter les blessures par piqûres, pour une seringue (2) comportant un corps de seringue (3) et un moyen piquant (5) disposé à l'extrémité distale (4) du corps de seringue (3), lequel dispositif de sécurité comporte un élément fourreau (6) s'étendant le long d'une direction axiale (X) et entourant au moins partiellement le moyen piquant (5) et le corps de seringue (3) et un élément intérieur (7) qui est disposé au moins par endroits à l'intérieur de l'élément fourreau (6) et qui entoure le moyen piquant (5),
dans lequel
l'élément intérieur (7) présente une partie formant col (8) et une partie (9) de protection du moyen piquant qui entoure le moyen piquant (5), la partie formant col (8) étant disposée sur une région d'extrémité distale (10) du corps de seringue (3) de façon tournante dans une direction périphérique (U), et fixant le dispositif de sécurité (1) sur le corps de seringue (3),
**caractérisé par le fait qu'**
une zone de rupture (11) est disposée entre la partie formant col (8) et la partie (9) de protection du moyen piquant, ce par quoi la partie (9) de protection du moyen piquant est détachable de la partie formant col (8) avant l'utilisation de la seringue (2), la partie formant col (8) présentant au moins une saillie de guidage (14), laquelle vient en prise dans au moins une coulisse de guidage (15) de l'élément fourreau (6) laquelle présente une trajectoire courbe, la zone de rupture (11) étant disposée entre une face frontale distale (12) de la partie formant col (8) et une extrémité proximale (13) de la partie (9) de protection du moyen piquant, la partie formant col (8) et la partie (9) de protection du moyen piquant formant d'un seul tenant l'élément intérieur (7).

2. Dispositif de sécurité (1) selon la revendication 1,
**caractérisé par le fait que**
la zone de rupture (11) est un amincissement de matériau et/ou une perforation et/ou une encoche et/ou une entaille dans le matériau.

3. Dispositif de sécurité (1) selon l'une des revendications précédentes,
**caractérisé par le fait que**
la saillie de guidage (14) est guidée dans ladite au moins une coulisse de guidage (15) de l'élément fourreau (6) lors d'un déplacement relatif du corps de seringue (3) par rapport à l'élément fourreau (6) sensiblement le long de la direction axiale (X).

4. Dispositif de sécurité (1) selon l'une des revendications précédentes,
**caractérisé par le fait que**
la partie (9) de protection du moyen piquant présente une cavité (17) dans une région d'extrémité proximale (16), le moyen piquant (5) et par endroits la région d'extrémité distale (10) du corps de seringue (3) étant aptes être disposés dans la cavité (17).

5. Dispositif de sécurité (1) selon la revendication 4,
**caractérisé par le fait qu'**
une partie intérieure en un matériau élastique est disposée dans la cavité (17) de la partie (9) de protection du moyen piquant, le matériau élastique étant du caoutchouc ou un élastomère synthétique.

6. Dispositif de sécurité (1) selon l'une des revendications précédentes,
**caractérisé par le fait que**
la partie formant col (8) est réalisée sensiblement en tant que cylindre à base circulaire creux (18), le cylindre à base circulaire (18) présentant une surface d'enveloppe (18a) sur laquelle est disposée ladite au moins une saillie de guidage (14), un diamètre extérieur (19) de la partie formant col (8) étant plus grand qu'un diamètre extérieur (20) de la partie (9) de protection du moyen piquant.

7. Dispositif de sécurité (1) selon l'une des revendications précédentes,
**caractérisé par le fait que**
la partie formant col (8) comporte une région distale (21) et une région proximale (22), la paroi de la partie formant col (8) présentant au moins deux fentes (23) dans la région distale (21), lesquelles s'étendent dans la direction axiale (X), et la région proximale (22) présentant un cône intérieur (24).

8. Dispositif de sécurité (1) selon l'une des revendications précédentes,
**caractérisé par le fait que**
le dispositif de sécurité (1) présente au moins un élément ressort (25) qui est relié fonctionnellement avec le corps de seringue (3) et s'oppose au déplacement relatif du corps de seringue (3) par rapport à l'élément fourreau (6).

9. Dispositif de sécurité (1) selon l'une des revendications précédentes,
**caractérisé par le fait que**
la partie formant col (8) et la partie (9) de protection du moyen piquant sont réalisées à partir de matériaux différents.

10. Dispositif de sécurité (1) selon l'une des revendications précédentes,
**caractérisé par le fait que**
la partie (9) de protection du moyen piquant est réalisée à partir d'un élastomère thermoplastique (TPE) et la partie formant col, à partir de polyoxyméthylène (POM).

11. Procédé de montage du dispositif de sécurité (1) selon la revendication 8 et d'agencement du dispositif de sécurité (1) sur un corps de seringue (3) par les étapes de procédé suivantes :
a) introduction de l'élément ressort (25) dans l'intérieur de l'élément fourreau (6) le long de la direction axiale (X) ;
b) introduction de l'élément intérieur (7) dans l'intérieur de l'élément fourreau (6), le long de la direction axiale (X) de telle sorte que l'élément ressort (25) est disposé entre la partie formant col (8) de l'élément intérieur (7) et l'élément fourreau (6) ;
c) application de la partie formant col (8) de l'élément intérieur (7) sur la région d'extrémité distale (10) du corps de seringue (3) ;
l'élément intérieur (7) étant chauffé lors du montage de telle sorte que celui-ci est déformable au moins partiellement.
